# EUROPÄISCHE PATENTSCHRIFT

(11) **EP 2 010 014 B1**
(45) Veröffentlichungstag und Bekanntmachung des Hinweises auf die Patenterteilung: **30.09.2009**
(21) Anmeldenummer: 07722747.8
(22) Anmeldetag: 08.01.2007
(51) Int. Cl.: A23L 3/3463, A23L 1/035

(54) **SOLUBILISATE VON KONSERVIERUNGSMITTELN SOWIE VERFAHREN ZU IHRER HERSTELLUNG**
SOLUBILIZATES OF PRESERVATIVES AND METHOD FOR PRODUCING THE SAME
SOLUBILISATS D'AGENTS DE CONSERVATION ET LEUR PROCÉDÉ DE PRODUCTION

(30) Priorität: 07.03.2006 DE 102006010809; 03.11.2006 WO PCT/EP2006/010568
(43) Veröffentlichungstag der Anmeldung: 07.01.2009
(73) Patentinhaber: Aquanova AG, 64295 Darmstadt (DE)
(72) Erfinder: BEHNAM, Dariush, 64380 Rossdorf (DE)
(74) Vertreter: Bill, Burkart Hartmut
(86) Internationale Anmeldenummer: PCT/EP2007/000094
(87) Internationale Veröffentlichungsnummer: WO 2007/101495

(56) Entgegenhaltungen:
- EP-A- 1 338 271
- EP-A- 1 475 083
- EP-A1- 0 572 190
- EP-A1- 0 659 347

## Beschreibung

Die Erfindung betrifft ein Solubililsat eines Konservierungsmittels.

In der deutschen Offenlegungsschrift 26 23 682 wird ein Verfahren zur Herstellung einer Suspension und einer Emulsion aus Sorbinsäure beschrieben, bei dem man eine heiße Lösung der Sorbinsäure in einem Lösungsmittel aus Wasser, versetzt mit einem wasserlöslichen niedrig siedenden organischen Lösungsmittel, versprüht, die erhaltene Flüssigkeit rasch abkühlt und die überschüssige Menge an Lösungsmittel entfernt. Allein schon durch die Erwärmung und das Versprühen der Sorbinsäurelösung ist dieses Verfahren sehr aufwendig.

In dem Dokument DE 25 19 557 A1 ist eine desinfizierende Seife beschrieben, welche neben dem Desinfektionsmittel und einem nicht-ionischen Emulgiermittel, wie beispielsweiche ein Polysorbat, einen erheblichen Anteil an Verdünnungsmittel sowie weitere Zusatzstoffe als Galenika enthält. Diese Seife dient zum Desinfizieren der Hände oder anderer Hautpartien im medizinischen Bereich.

Die Schrift DE 601 03 023 T2 offenbart saure antimikrobielle Zusammensetzungen zur Behandlung von Lebensmitteln, welche eine organische Säure, ein gegebenenfalls nicht-ionisches Tensid und ein Stabilisierungsmittel, beispielsweise ein Polysorbat, umfasst. In erheblicher Verdünnung der Zusammensetzung kann sie zur Behandlung von Nahrungsmittel-Oberflächen dienen, um deren Mikroorganismenbesatz zu reduzieren.

Zur Haltbarmachung von Lebensmitteln gegen mikrobiellen Verderb gibt es neben der biologischen und chemischen Säuerung, sowie Erhitzungsprozessen u.a. die chemische Konservierung mit bakteriziden bzw. bakteriostatischen Chemikalien.

Die für Getränke gebräuchlichsten Konservierungsmittel sind Sorbinsäure (E200 und Salze E201-3) und Benzoesäure (E210 und Salze E211-13).

Benzoesäure (BS) wirkt hauptsächlich gegen Hefen und Schimmelpilze, Bakterien werden nur teilweise gehemmt. Sorbinsäure (SS ) wirkt ebenfalls gegen Hefen und Schimmelpilze, jedoch etwas besser auch gegen Bakterien, vor allem gegen katalasepositive und anaerobe Bakterien. In der Praxis hat sich eine Mischkonservierung aus gleichen Teilen Benzoesäure und Sorbinsäure mit einer gewissen synergistischen Wirkung bewährt. Hiermit gelingt es auch, die Geschmacksbeeinträchtigung des Lebensmittels durch zu hohe Konservierungsmengen zu reduzieren. Übliche Einsatzmengen sind 150 - 200 ppm je Konservierungsstoff im Fertigprodukt. Die Höchstmengen bei alkoholfreien Getränken sind auf 300 ppm SS bzw. 150 ppm BS begrenzt, im Gemisch sind die Höchstmengen 250 ppm SS + 150 ppm BS.

Diese Konservierungsstoffe werden bei Getränken und anderen stark wasserhaltigen flüssigen Lebensmitteln allgemein in der Form ihrer gut wasserlöslichen Natrium-, Kalium- und Calciumsalze eingesetzt. Die bakterizide Wirkung entwickeln jedoch ausschließlich die undissoziierten Säuren. Enthalten die mit Benzoesäure und/oder Sorbinsäure zu konservierenden Lebensmittel Ascorbinsäure, besteht zudem die Gefahr, dass durch eine Reaktion der Ascorbinsäure mit der Benzoesäure und/oder der Sorbinsäure unerwünschte weil gesundheitsschädliche Reaktionsprodukte wie etwa Benzol, entstehen können.

Die Löslichkeiten der genannten Konservierungsmittel in Wasser, Ethanol und Öl betragen:

| Stoff | Wasser | 100 % Ethanol | Pflanzenöl |
|---|---|---|---|
| Sorbinsäure | 0,16 g/100 g | 13 g/100 g | 1 g/100 g |
| Kaliumsorbat | 138 g/100 g | n.a. | n.a. |
| Benzoesäure | 0,34 g/100 g | gut löslich | 2 g/100 g |
| Natriumbenzoat | 63 g/100 g | n.a. | n.a. |

Die Dissoziation der schwachen Säuren SS (pKs = 4,8) und BS (pKs = 4,22) ist stark vom pH-Wert des Mediums abhängig, in welchem sie eingesetzt werden. Bei pH-Werten, wie sie für klassische alkoholfreie Getränke üblich sind (pH = 2,8 - 3,5), sind 90 - 100 % von SS oder BS undissoziiert und damit bakterizid wirksam. Hier ist jedoch durch die schlechte Wasserlöslichkeit der Säuren eine relativ hohe Dosis notwendig. In wenig sauren Produkten dagegen (pH-Werte 4,0 - neutral) sind, vor allem bei der BS, beträchtliche Anteile dissoziiert, die keine Wirkung gegen Mikroorganismen haben.

| PH-Wert | 3 | 3,5 | 4,0 | 5,0 |
|---|---|---|---|---|
| BS-Anion, diss. | 6% | 16,7 % | 38,7 % | 86,5 % |
| SS-Anion, diss. | 1,7 % | | 14,7 % | 63 % |

Der Erfindung liegt daher die Aufgabe, Lebensmittel, vornehmlich Getränke, gegen den Befall von Mikroorganismen besser zu schützen.

Dazu schlägt die Erfindung ein stabilisierungsmittelfreies Solubilisat eines Konservierungsmittels gemäß Anspruch 1 vor. Zweckmäßig ist der Emulgator ein Polysorbat, bevorzugt Polysorbat 20 und/oder Polysorbat 80.

Das erfindungsgemäße Solubilisat hat eine ausgezeichnete bakterizide Wirkung. Gibt man beispielsweise zu einer Nährlösung Agar Agar einerseits 500 mg Kaliumsorbat und andererseits im Vergleich 500 mg Sorbinsäure-Solubilisat mit Polysorbat 20, erhält man nach etwa drei Wochen Einwirkzeit mit Kaliumsorbat eine Keimzahl von 1,6 x 10¹⁰, dagegen mit Sorbinsäure-Solubilisat eine Keimzahl von 3,5 x 10⁸, also eine um fast zwei Zehnerpotenzen kleinere Keimzahl.

Der Erfindung liegt die Konzeption zugrunde, eine Dissoziierung der organischen Säuren vor allem in nur schwach sauren Medien durch Mizellierung zu verlangsamen oder zu verhindern mit der Folge, dass zur Abtötung von Mikroorganismen mehr undissoziierte Säure zur Verfügung steht. Daher kann die Dosis der micellierten organischen Säuren deutlich herabgesetzt werden. Da sich die Mizellen nur bei Kontakt mit Zellmembranen oder ähnlichem biologischen Material öffnen, kann die aus den Mizellen entlassene Säure direkt auf die Mikroorganismen abtötend einwirken.

Die Erfindung kann verwendet werden für Lebensmittel aller Art, insbesondere in nichtalkoholischen Getränken mit hohem pH-Wert von zum Beispiel etwa 4,0 bis etwa 7,0, wie Tee-, Kaffee- Kakao- und Milchgetränke. Die Konservierungsstoffsolubilisate werden den jeweiligen Tee-, Kaffee- Kakao- und Milchgetränken zugesetzt. Die Dosierung erfolgt individuell durch den Hersteller des Endproduktes zwischen 100 ppm und 500 ppm, das bedeutet, ausgehend von einem zwanzigprozentigen Benzoesäuresolubilisat zwischen 500 ppm und 1000 ppm pro Liter Endprodukt.

Die Erfindung kann ferner für die Konservierung von Milchprodukten wie Milch, Käse und Joghurt verwendet werden. Die Solubilisate werden hauptsächlich auf die Käserinde aufgetragen. Das Sorbinsäuresolubilisat darf auch in geringer Menge im Käse eingesetzt werden. Darüber hinaus können Gemüse, Früchte und Kräuter in eine wässrige Lösung des erfindungsgemäßen Solubilisats eingetaucht und dadurch gegen Schimmel, Pilze und Hefe besser geschützt und damit haltbarer gemacht werden. Schließlich erlaubt die Erfindung auch eine Verwendung von Konservierung von Oberflächen von Holz und/oder Kunststoffen, so dass beispielsweise Möbel oder die Oberflächen medizinischer Apparate und Geräte zur Konservierung mit erfindungsgemäßen Solubilisaten in verdünnter wässriger Lösung behandelt werden können.

Die bakterizide Wirkung beispielsweise eines Benzoesäuresolubilisats geht aus einer Untersuchung eines öffentlich bestellten und vereidigten Sachverständigen für Lebensmittelchemie hervor.

### Testmaterialien:

1) Benzoesäure: Rohstoff Benzoesäure, Firma Merck Art.Nr. 1.00136
2) Benzoesäuresolibilisatlösung: Rohstoff 20%iges Benzoesäuresolubilisat, Firma AQUANOVA Lot-Nr. L096.06.LM.01.01
3) Nährlösung: Yeast Malt Broth, Firma Sigma Art.Nr. Y3752
4) Nähragar: siehe 3) mit Zusatz von Agar, Firma Fluka Art.Nr.05040, 20g pro Liter Nährlösung
5) Hefekultur: Saccharomyces cerevisiae, Firma DSMZ Art.Nr.7044

Die Zusammensetzung des 20%iges Benzoesäuresolubilisats nach 2) ist in den nachfolgenden Beispielen unter EW0108/8 angegeben.

Die nachfolgende Tabelle 1 zeigt den Einfluß von Benzoesäure auf das Hefewachstum und die Tabelle 2 gibt die erreichte Hemmung des Hefewachstums, jeweils bei pH 6,2 und Zimmertemperatur wieder. Aus Tabelle 1 ergibt sich, dass selbst bei einer Benzoesäurekonzentration von 500 ppm auch nach 29 Tagen Einwirkzeit ein immer noch hohes Hefewachstum festzustellen ist. Dagegen sieht man aus der Tabelle 2, dass bei einer Benzoesäurekonzentration von 500 ppm im Solubilisat das Hefewachstum von Beginn der Solubilisatzugabe an deutlich gebremst ist und nach 29 Tagen Einwirkzeit die Hefen völlig abgetötet sind. Schon nach dem dritten Tag der Einwirkung führt eine Benzoesäurekonzentration im Solubilisat von 300 ppm zu einer fortschreitenden Wachstumshemmung der Hefe.

Bevorzugte Ausführungsformen der erfindungsgemäßen Solubilisate sind in den Unteransprüchen angegeben. So empfiehlt es sich, dass das Sorbinsäuresolubilisat etwa 5 Gew% Sorbinsäure und etwa 95 Gew% Polysorbat 20 aufweist. Für die Benzoesäure ist ein Solubilisat zweckmäßig, das etwa 20 Gew% Benzoesäure und etwa 80 Gew% Polysorbat 20 enthält. Weiterhin ist es für viele Verwendungsfälle, beispielsweise für einen Zusatz zu angesäuerten Getränken zweckmäßig, wenn das Benzoesäuresolubilisat einen Anteil eines Netzmittels enthält, welches die micellierte Benzoesäure soweit bindet, dass im Getränk praktisch keine freie Benzoesäure mehr vorhanden ist. Ist das Getränk etwa mit Ascorbinsäure versetzt, kann aus der Einwirkung von Ascorbinsäure auf Benzoesäure leicht das lebensmittelschädliche Benzol entstehen. Das erfindungsgemäße Benzoesäuresolubilisat verhindert also eine Benzolbildung in angesäuerten Getränken. Das Netzmittel umfasst zweckmäßig eine Mischung aus einem vorwiegend mittelkettige Triglyceride enthaltenden Öl mit einem geringen Anteil an Wachs, beispielsweise Cera alba.

Das Netzmittel kann in dem Benzoesäuresolubilisat mit einem Gehalt von etwa 2 Gew% bis etwa 10 Gew% vertreten sein, wobei auf das Öl etwa 3 Gew% bis etwa 10 Gew% auf das Wachs etwa 1 Gew% bis etwa 2 Gew% entfallen können.

Im Einzelnen erweist sich ein Benzoesolubilisat in seiner bakteriziden Wirkung als besonders günstig, wenn es etwa 21 Gew% Benzoesäure, etwa 5 Gew% eines vorwiegend mittelkettige Triglyceride enthaltenden Öls, etwa 73 Gew% Polysorbat 20 und etwa 1 Gew% eines Wachses wie beispielsweise Bienenwachs (Cera alba) enthält. Statt des Polysorbats 20 kann auch Polysorbat 80 in etwa gleicher Menge für das Benzoesäuresolibilisat eingesetzt werden. Schließlich kommt auch ein Solubilisat in Betracht, welches aus etwa 5 Gew% Sorbinsäure, etwa 5 Gew% Benzoesäure und etwa 90 Gew% Polysorbat 20 besteht.

### Benzoesäure (Einwaage Benzoesäure + 50 ml Nährlösung (pH 6,2) + 50 ml Wasser + 0,4 ml Hefelösung)

| Benzoesäurekonzentration | 10 ppm | 50 ppm | 100 ppm | 200 ppm | 300 ppm | 500 ppm |
|---|---|---|---|---|---|---|
| Ausgangskeimzahl | 2.900 /ml | 2.900 ml | 2.900 /ml | 2.900 /ml | 2.900 /ml | 2.900 /ml |
| nach 3-Tagen | 1.650.000 /ml | 1.900.000 /ml | 670.000 /ml | 510.000 /ml | 630.000 /ml | 700 /ml |
| nach 7 Tagen | 1.200.000 /ml | 1.100.000 /ml | 530.000 /ml | 430.000 /ml | 400.000 /ml | 55.000 /ml |
| nach 15 Tagen | 800.000 /ml | 900.000 /ml | 600.000 /ml | 350.000 /ml | 520.000 /ml | 230.000 /ml |
| nach 22 Tagen | 1.100.000 /ml | 930.000 /ml | 1.200.000 /ml | 400.000 /ml | 250.000 /ml | 120.000 /ml |
| nach 29 Tagen | 1.300.000 /ml | 1.300.000 /ml | 900.000 /ml | - 400.000 /ml | 230.000 /ml | 43.000 /ml |

### 2.) Benzoesäure-Solubilisat (Einwaage Solubilisat + 50 ml Nährlösung (pH 6,2) + 50 ml Wasser + 0,1 ml Hefelösung)

| Solubilisatzugabe | 50 ppm | 250 ppm | 500 ppm | 1000 ppm | 1500 ppm | 2500 ppm |
|---|---|---|---|---|---|---|
| Benzoesäurekonzentration | 10 ppm | 50 ppm | 100 ppm | 200 ppm | 300 ppm | 500 ppm |
| Ausgangskeimzahl | 5.500 /ml | 5.500 /ml | 5.500 /ml | 5.500 /ml | 5.500 /ml | 5.500 /ml |
| nach 3 Tagen 28.04.06 | 1.990.000 /ml | 4.170.000 /ml | 800.000 /ml | 235.000 /ml | 229.000 /ml | 1.520 /ml |
| nach 9 Tagen 4.05.06 | 790.000 /ml | 3.100.000 /ml | 630.000 /ml | 360.000 /ml | 185.000 /ml | 500 /ml |
| nach 15 Tagen 10.05.06 | 2.250.000 /ml | 815.000 /ml | 85.000 /ml | 65.000 /ml | 37.000 /ml | 15 /ml |
| nach 22 Tagen 17.05.06 | 4.325.000 /ml | 750.000 /ml | 50.000 /ml | 35.000 /ml | 12.000 /ml | n.n. in 1 ml |

Nachfolgend werden fünf spezielle Rezepturen ohne Beschränkung der Allgemeinheit der Patentansprüche mitgeteilt, die auch Angaben über die jeweilige Art der Herstellung der einzelnen Solubilisate enthalten. In der einfachsten Form des Herstellungsverfahren gestaltet sich dieses in der Weise, dass zunächst der Emulgator auf etwa 70°C bis etwa 90°C, im Falle des Polysorbats 20 auf etwa 72°C bis etwa 85°C, bevorzugt auf etwa 80°C bis etwa 85°C erwärmt wird, die aliphatische und/oder die aromatische Säure langsam, das heißt ohne wesentliche (höchstens 5 °C) Abkühlung des Emulgators, unter Rühren in den warmen Emulgator eingearbeitet wird, nach vollständiger Einarbeitung die Mischung unter ständigem Rühren auf etwa 83°C bis etwa 90°C weiter erwärmt und gut homogenisiert wird. Danach lässt man das Solubilisat zum Abfüllen auf unter 35°C abkühlen, wobei sich ein rascher Abkühlungsschritt auf etwa 40°C empfiehlt. Zweckmäßig werden in dem Herstellungsverfahren etwa 50 Gew% bis etwa 95 Gew% an Emulgator eingesetzt, der beispielsweise Polysorbat 20 und/oder Polysorbat 80 sein kann. Für die aliphatische und/oder aromatische Säure empfiehlen sich etwa 5 Gew% bis etwa 50 Gew% zu verwenden.

Wenn das Benzoesäuresolubilisat ein Netzmittel enthalten soll, geht man zweckmäßig so vor, dass zunächst das Netzmittel auf etwa 55°C bis etwa 65°C, bevorzugt 58°C bis etwa 62°C erwärmt und homogenisiert wird, dass dem warmen Netzmittel die Benzoesäure hinzugegeben und erneut unter Rühren homogenisiert wird, dass danach etwa ein Fünftel der Polysorbatmenge, also etwa 150g pro kg Solubilisat, dem Benzoesäure/Netzmittel-Gemisch zugegeben und die erhaltene Masse unter Rühren auf etwa 80°C bis etwa 90°C erwärmt und homogenisiert wird und dass danach die restliche Polysorbatmenge, also vier Fünftel derselben, langsam dazugegeben, gut gerührt und die Temperatur wenigstens 5 Minuten bei etwa 80°C bis etwa 90°C gehalten wird. Danach kann gegebenenfalls rasch auf etwa 40°C abgekühlt und bei etwa 35°C abgefüllt werden. Bei diesem Verfahren können etwa 2 Gew% bis etwa 10 Gew% an Netzmittel eingesetzt werden. Wenn in bevorzugter Ausgestaltung des Verfahrens als Netzmittel eine Mischung aus einem vorwiegend mittelkettige Triglyceride enthaltendem Öl und einem Wachs benutzt wird, ist es zweckmäßig, in etwa 5 Gew% des wie angegeben erwärmten Öls etwa 1 Gew% bis 1,5 Gew% des Wachses einzuarbeiten und die Mischung zu homogenisieren und anschließend etwa 20 Gew% Benzoesäure hinzugeben und danach wie vorstehend angegeben das Polysorbat hinzufügen. Im Übrigen sind bevorzugte Ausführungsformen des erfindungsgemäßen Verfahrens in den Verfahrensansprüchen angegeben.

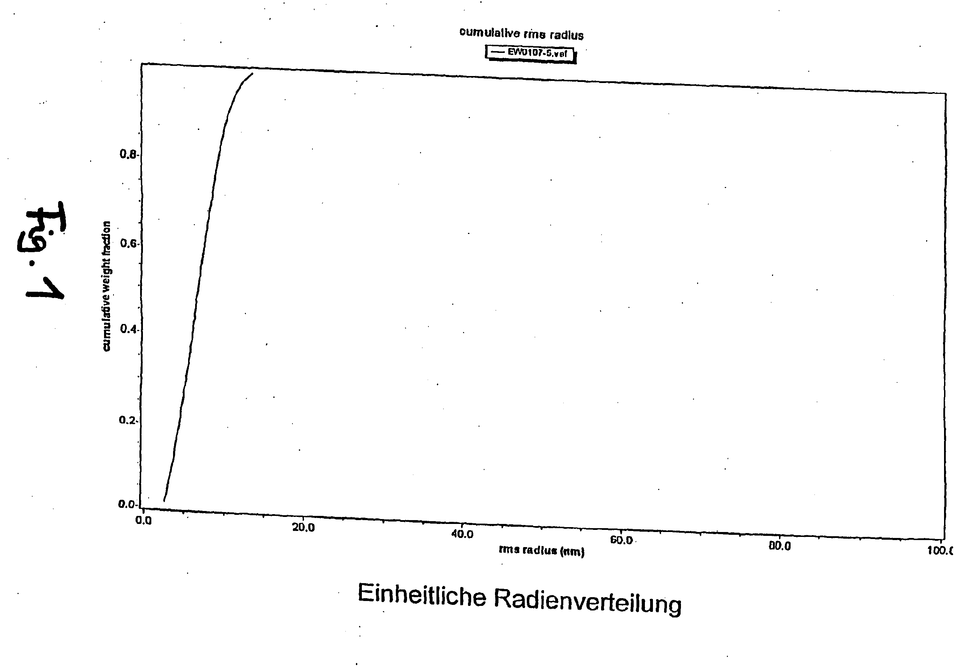

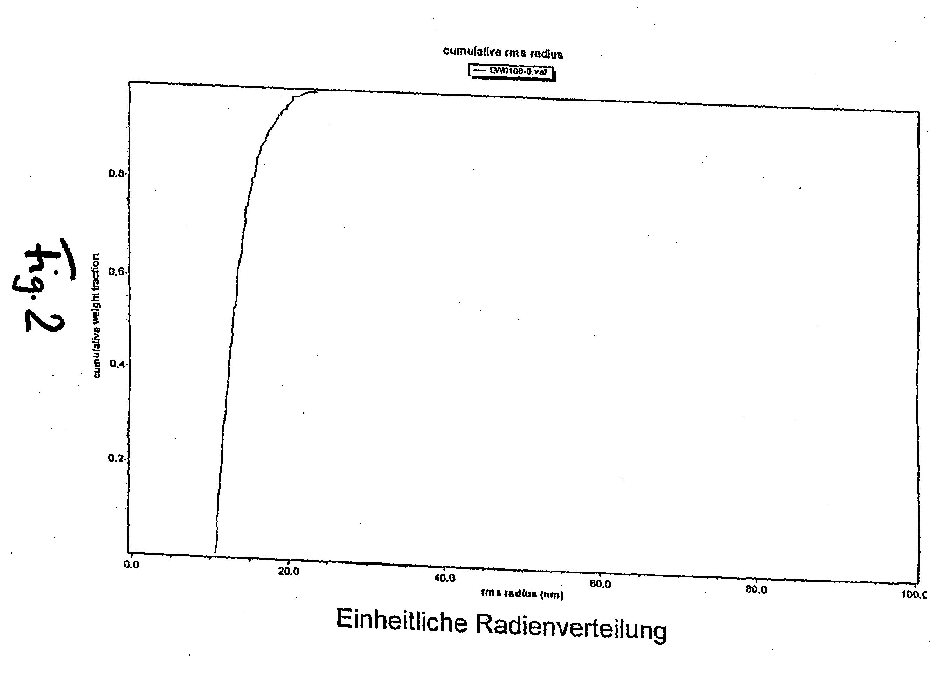

Für die erfindungsgemäßen Solubilisate sind die Radiusverteilungen der gebildeten Micellen gemessen worden. Das Ergebnis zeigen die beigefügten Figuren 1 und 2. Die Messungen wurden nach der Feldfluß-Methode der Wyatt Technologie Europe GmbH durchgeführt. Figur 1 bezieht sich auf das 5 %-ige Sorbinsäure-Solubilisat und Figur 2 gilt für das 20 %-ige Benzoesäure-Solubilisat. Man erkennt, dass der mittlere Micellenradius für das Sorbinsäuresolubilisat unter 10 nm und der mittlere Micellenradius für das Benzoesäuresolubilisat unter 20 nm liegt.

In den Rezepturen bedeutet MCT medium chain triglyceride, also mittelkettiges Triglycerid. Die Gewichtsangaben in Prozent beziehen sich auf das Gesamtgewicht des Solubilisats = 100Gew%.

| | |
|---|---|
| EW-Nr.: | EW0108/8 |
| Bezeichung : | Wasser-und fettlösliches 20%iges Benzoesäure-Solubilisat |

### Zutaten:

| | |
|---|---|
| 200 g | Benzoesäure; AppliChem (AGT-Material-Nr.: 10080/079) |
| 800 g | Polysorbat 20; Lamesorb SML 20; Cognis (AGT-Material-Nr.: 10520/016) |

### Durchführung:

- Polysorbat 20 auf 80 -85°C erwärmen.
- Benzoesäure langsam, unter Rühren einarbeiten.
- Alles unter Rühren auf max. 90°C erhitzen und gut homogenisieren.
- Abkühlen lassen auf < 35°C und abfüllen.

### Aussehen:

Hellgelb, viskos, transparent

### Lagerbedingungen:

Dunkel, bei Raumtemperatur (< 25°C)

| | |
|---|---|
| EW-Nr.: | EW0108/80/CA |
| Bezeichung: | NovaSOL^{®} Benzoic Wasser-und fettlösliches 20% iges Benzoesäure-Solubilisat |

### Zutaten:

| | |
|---|---|
| 210 g | Benzoesäure; AppliChem (AGT-Material-Nr.: 10080/079) |
| 50 g | Delios VK koscher (MCT-Öl): Cognis (AGT-Material-Nr.: 10460/016) |
| 739 g | Polysorbat 80; Lamesorb SMO 20; Cognis (AGT-Material-Nr.: 10530/016) |
| 1 g | Cera alba (Bienenwachs); Roeper (AGT-Material-Nr.: 10741/075) |

### Durchführung:

- MCT-Öl erwärmen (58-62°C) und Cera alba langsam, unter Rühren einarbeiten und homogen mischen.
- Dem Gemisch Benzoesäure hinzugeben und unter Rühren gut homogenisieren (58-62°C)
- Etwa 20 % der Polysorbat 80-Menge (ca. 150g/kg) zur Mischung hinzugeben und alles unter Rühren auf 83-87°C erhitzen bis die Mischung homogen ist.
- Restliche Polysorbat 80-Menge langsam dazugeben, gut rühren und die Temperatur dabei mindestens 5 Minuten bei 83-87°C halten.
- Unter ständigem Rühren schnellstmälich auf 40°C abkühlen
- Abfüllung ab 40°C.

### Aussehen:

Hellgelb/weiß, viskos

### Lagerbedingungen:

Dunkel, bei Raumtemperatur (< 25°C)

| | |
|---|---|
| EW-Nr.: | EW0109/12 |
| Bezeichung: | Wasser-und fettlösliches 5% Sorbinsäure / 5 % Benzoesäure-Solubilisat (Kombination) |

### Zutaten:

| | |
|---|---|
| 50 g | Sorbinsäure; Krämer & Martin (AGT-Matenal-Nr.: 10642/088) |
| 50 g | Benzoesäure; AppliChem (AGT-Material-Nr.: 10080/079) |
| 900 g | Polysorbat 20; Lamesorb SML 20; Cognis (AGT-Material-Nr.: 10520/016) |

### Durchführung:

- Sorbinsäure und Benzoesäure mischen.
- Polysorbat 20 auf 80-85°C erwärmen
- Pulvergemisch langsam unter Rühren ins Polysorbat einarbeiten (83-87°C).
- Alles unter Rühren auf ca. 88-92°C erhitzen und gut homogenisieren.
- Abkühlen lassen auf < 35°C und abfüllen.

### Aussehen:

Gelb, viskos, transparent

### Lagerbedingungen:

Dunkel, bei Raumtemperatur (< 25°C)

| | |
|---|---|
| EW-Nr.: | EW0108/CA/1 |
| Bezeichung: | NovaSOL^{®} Benzoic Wasser- und fettlösliches 20%iges Benzoesäure-Solubilisat |

### Zutaten:

| | |
|---|---|
| 210 g | Benzoesäure; AppliChem (AGT-Material-Nr.: 10080/079) |
| 50 g | Delios VK koscher (MCT-Öl); Cognis (AGT-Material-Nr.: 10460/016) |
| 738,5 g | Polysorbat 20; Lamesorb SML 20; Cognis (AGT-Material-Nr.: 10520/016) |
| 1,5 g | Cera alba (Bienenwachs); Roeper (AGT-Material-Nr.: 10741/075) |

### Durchführung:

- MCT-Öl erwärmen (58-62°C) und Cera alba langsam, unter Rühren einarbeiten und homogen mischen.
- Dem Gemisch Benzoesäure hinzugeben und unter Rühren gut homogenisieren (58-62°C)
- Etwa 20 % der Polysorbat 20-Menge (ca. 150g/kg) zur Mischung hinzugeben und alles unter Rühren auf 83-87°C erhitzen bis die Mischung homogen ist.
- Restliche Polysorbat 20-Menge langsam dazugeben, gut rühren und die Temperatur dabei mindestens 5 Minuten bei 83-87°C halten.
- Unter ständigem Rühren schnellstmöglich auf 40°C abkühlen
- Abfüllung ab 40°C.

### Aussehen:

Hellgelb/weiß, viskos

### Lagerbedingungen:

Dunkel, bei Raumtemperatur (< 25°C)

| | |
|---|---|
| EW-Nr.: | EW0107/5 |
| Bezeichung: | Wasser- und fettlösliches 5%iges Sorbinsäure-Solubilisat |

### Zutaten:

| | |
|---|---|
| 50 g | Sorbinsäure; AppliChem (AGT-Material-Nr.: 10641/079) |
| 950 g | Polysorbat 20; Lamesorb SML 20; Cognis (AGT-Material-Nr.: 10520/016) |

### Durchführung:

- Polysorbat 20 auf 72 -77°C erwärmen.
- Sorbinsäure langsam, unter Rühren einarbeiten.
- Alles unter Rühren auf max. 90°C erhitzen und gut homogenisieren.
- Abkühlen lassen auf < 35°C und abfüllen.

### Aussehen:

Hellgelb, viskos, transparent

### Lagerbedingungen:

Dunkel, bei Raumtemperatur (< 25°C)

## Patentansprüche

1. Stabilisierungsmittelfreies Solubilisat eines Konservierungsmittels zur Haltbarmachung von Lebensmitteln gegen mikrobiellen Verderb enthaltend eine aliphatische und/oder aromatische Säure wie beispielsweise Sorbinsäure und/oder Benzoesäure, sowie einen oder mehrere Emulgatoren mit einem HLB-Wert zwischen 9 und 18 mit einem Gehalt zwischen etwa 50 Gew% und etwa 95 Gew% an Emulgator, bezogen auf die Gesamtmenge des Solubilisats, wobei die Säure mizelliert vorliegt, so daß die aus den Mizellen entlassene Säure direkt auf die Mikroorganismen abtötend einwirken kann, wenn sich die Mizellen bei Kontakt mit Zellmembranen oder ähnlichen biologischen Material öffnen.

2. Solubilisat nach Anspruch 1 mit einem Gehalt zwischen 5 Gew% und 50 Gew% an aliphatischer und/oder aromatischer Säure.

3. Solubilisat nach Anspruch 1 oder 2, bei welchem der Emulgator ein Polysorbat, vornehmlich Polysorbat 20 und/oder Polysorbat 80 ist.

4. Solubilisat nach einem der vorstehenden Ansprüche bestehend aus etwa 5 Gew% Sorbinsäure und etwa 95 Gew% Polysorbat 20.

5. Solubilisat nach einem der Ansprüche 1 bis 4 bestehend aus etwa 20 Gew% Benzoesäure und etwa 80 Gew% Polysorbat 20.

6. Solubilisat nach einem der Ansprüche 1 bis 4 bestehend aus etwa 20 Gew% Benzoesäure, etwa 5 Gew% eines vorwiegend mittelkettige Triglyceride enthaltenden Öls, etwa 73,5 Gew% Polysorbat 20 und etwa 1,5 Gew% eines Wachses.

7. Solubilisat nach Anspruch 6 bei dem das Wachs Bienenwachs etwa in der Form von Cera alba ist.

8. Solubilisat nach Anspruch 6 oder 7, bei welchem das Polysorbat 20 durch Polysorbat 80 ersetzt ist.

9. Solubilisat nach einem der Ansprüche 1 bis 3 bestehend aus etwa 5 Gew% Sorbinsäure, etwa 5 Gew% Benzoesäure und etwa 90 Gew% Polysorbat 20.

10. Verfahren zur Herstellung eines Solubilisats nach einem oder mehreren der vorstehenden Ansprüche, bei welchem ein Emulgator mit einem HLB-Wert von 9 - 18 auf etwa 70°C bis etwa 90°C erwärmt wird, die aliphatische und/oder aromatische Säure ohne wesentliche Abkühlung des Emulgators unter Rühren in den warmen Emulgator eingearbeitet wird, nach vollständiger Einarbeitung die Mischung unter ständigem Rühren auf etwa 83°C bis etwa 90°C erwärmt und gut homogenisiert und anschließend abgekühlt wird, so daß die Dissoziierung der organischen Säure oder Säuren durch Mizellierung verlangsamt oder verhindert wird.

11. Verfahren nach Anspruch 10, bei welchem etwa 50 Gew% bis etwa 95 Gew% an Emulgator erwärmt werden.

12. Verfahren nach Anspruch 10 oder 11, bei welchem ein Polysorbat, vornehmlich Polysorbat 20 und/oder Polysorbat 80 als Emulgator eingesetzt wird.

13. Verfahren nach Anspruch 12, bei welchem das Polysorbat auf etwa 72°C bis etwa 85°C, bevorzugt auf etwa 80°C bis etwa 85°C erwärmt wird.

14. Verfahren nach einem der Ansprüche 10 bis 13, bei welchem etwa 5 Gew% bis etwa 50 Gew% an aliphatischer und/oder aromatischer Säure in den warmen Emulgator eingearbeitet werden.

15. Verfahren nach einem der Ansprüche 10 bis 14, bei welchem rasch auf etwa 40 °C abgekühlt wird.

16. Verfahren nach einem der Ansprüche 10 bis 15, bei welchem Benzoesäure einem warmen und homogenisierten Netzmittel hinzugegeben und die Mischung homogenisiert wird, dass danach etwa ein Fünftel der Polysorbatmenge dem Benzoesäure/Netzmittelgemisch zugegeben und die erhaltene Masse unter Rühren erwärmt und homogenisiert wird und anschließend die restliche Polysorbatmenge langsam hinzugefügt und bei erhöhter Temperatur gerührt wird.

17. Verfahren nach Anspruch 16, bei welchem das Netzmittel auf etwa 55°C bis etwa 65°c, bevorzugt 58°C bis etwa 62°C erwärmt wird.

18. Verfahren nach Anspruch 15 oder 16, bei welchem die erhöhte Temperatur zu etwa 80°C bis etwa 90°C gewählt wird.

19. Verfahren nach einem der Ansprüche 16 bis 19, bei welchem als Netzmittel eine Mischung aus einem vorwiegend mittelkettige Triglyceride enthaltenden Öl und einem Wachs, bevorzugt Cera alba gewählt wird.

20. Verfahren nach Anspruch 19, bei welchem das Netzmittel aus etwa 5 Gew% des Öles und etwa 1 Gew% bis etwa 1,5 Gew% des Wachses gemischt wird.

21. Verwendung eines stabilisierungsmittelfreien Solubilisats nach einem der Ansprüche 1 bis 9, als konservierender Zusatz zu Lebensmitteln, insbesondere nichtalkoholischen Getränken mit hohem pH-Wert von zum Beispiel etwa 4,0 bis etwa 7,0, wie Tee-, Kaffee-, Kakao- und Milchgetränke.

22. Verwendung nach Anspruch 21 mit einer Dosierung zwischen 500 ppm und 1000 ppm pro Liter Endprodukt.

23. Verwendung eines stabilisierungsmittelfreien Solubilisats nach einem der Ansprüche 1 bis 9 als konservierender Zusatz zu Milchprodukten wie Milch,Käse oder Joghurt, wobei das Solubilisat hauptsächlich auf Käserinde aufgetragen wird.

24. Verwendung eines stabilisierungsmittelfreien Solubilisats nach einem der Ansprüche 1 bis 9 zur Konservierung von Gemüse, Früchten und Kräutern zweckmäßig durch Eintauchen von Gemüse oder Früchten oder Kräutern in eine wässrige Lösung des Solubilisats.

25. Verwendung eines stabilisierungsmittelfreien Solubilisats nach einem der Ansprüche 1 bis 9 zur Konservierung von Oberflächen von Holz oder Kunststoffen wie etwa Möbel, oder von Oberflächen medizinischer Apparate und Geräte durch Behandeln mit verdünnter wässriger Lösung des Solubilisats.

## Claims

1. Stabilising agent-free solubilizate of a preservative for preserving foodstuffs against microbial spoilage, containing an aliphatic and/or aromatic acid, e.g., sorbic acid and/or benzoic acid, as well as one or more emulsifiers having an HLB value between 9 and 18 having an emulsifier content between approximately 50 wt.% *BXX*. approximately 95 wt.% with respect to the total amount of the solubilizate, wherein the acid is present in micelle-form so that the acid released from the micelles can acv directly on the micro-organisms in a deadly manner when the micelles open upon contact with cell membranes or similar biological material.

2. Solubilizate as claimed in Claim 1, having an aliphatic and/or aromatic acid content between 5 wt.% and 50 wt.%.

3. Solubilizate as claimed in Claim 1 or 2, wherein me emulsifier is a polysorbate, preferably polysorbate 20 and/or polysorbate 80.

4. Solubilizate as claimed in any one of the preceding Claims, consisting of approximately 5 wt.% sorbic acid and approximately 95 wt.% polysorbate 20.

5. Solubilizate as claimed in any one of Claims 1 to 4, consisting of approximately 20 wt.% benzoic acid and approximately 80 wt.% polysorbate 20.

6. Solubilizate as claimed in any one of Claims 1 to 4, consisting of approximately 20 wt.% benzoic acid, approximately 5 wt.% of an oil predominantly containing medium-chained triglycerides, approximately 73.5 wt.% polysorbate 20 and approximately 1.5 wt.% of a wax.

7. Solubilizate as claimed in Claim 6, wherein the wax is beeswax, e.g., in the form of cera alba.

8. Solubilizate as claimed in Claim 6 or 7, wherein the polysorbate 20 isireplaced by polysorbate 80.

9. Solubilizate as claimed in any one of Claims 1 to 3, consisting of approximately 5 wt.% sorbic acid, approximately 5 wt.% benzoic acid and approximately 90 wt.% polysorbate 20.

10. Method of producing a solubilizate as claimed in any one or several of the preceding Claims, wherein an emulsifier having an HLB value of 9-18 is heated to approximately 70°C to approximately 90°C, the aliphatic and/or aromatic acid is incorporated under stirring into the hot emulsifier without substantial cooling of the emulsifier, when incorporation has been completed the mixture is heated, whilst being constantly stirred, to approximately 83°C to approximately 90°C and is effectively homogenised and then cooled so that the dissociation of the organic acid(s) by micelle formation is retarded or prevented.

11. Method as claimed in Claim 10, wherein approximately 50 wt.% to approximately 95 wt.% of the emulsifier is heated.

12. Method as claimed in Claim 10 or 11, wherein a polysorbate, preferably polysorbate 20 and/or polysorbate 80 is used as the emulsifier.

13. Method as claimed in Claim 12, wherein the polysorbate is heated to approximately 72°C to approximately 85°C, preferably to approximately 80°C to approximately 85°C.

14. Method as claimed in any one of Claims 10 to 13, wherein approximately 5 wt.% to approximately 50 wt.% of the aliphatic and/or aromatic acid is incorporated into the hot emulsifier.

15. Method as claimed in any one of Claims 10 to 14, wherein cooling to approximately 40°C is quickly effected.

16. Method as claimed in any one of Claims 10 to 15, wherein benzoic acid is added to a hot and homogenised wetting agent and the mixture is homogenised, in that subsequently approximately one fifth of the amount of polysorbate is added to :k benzoic acid/wetting agent mixture and the remaining mass is heated and homogenised whilst being stirred and then the remaining amount of polysorbate is slowly added and stirred at an increased temperature.

17. Method as claimed in Claim 16, wherein the wetting agent is heated to approximately 55°C to approximately 65°C, preferably 58°C to approximately 62°C.

18. Method as claimed in Claim 15 or 16, wherein the increased temperature is selected to be approximately 80°C to approximately 90°C.

19. Method as claimed in any one of Claims 16 to 18, wherein a mixture consisting of an oil, predominantly containing medium-chained triglycerides, and a wax, preferably cera alba, is selected as the wetting agent.

20. Method as claimed in Claim 19, wherein the wetting agent consisting of approximately 5 wt.% of the oil and approximately 1 wt.% to approximately 1.5 wt.% of the wax is mixed.

21. Use of a stabilising agent-free solubilizate as claimed in any one of Claims 1 to 9 as a preserving additive in foodstuffs, in particular non-alcoholic drinks having a high pH value of for example approximately 4.0 to approximately 7.0, such as tea, coffee, cocoa and milk drinks.

22. Use as claimed in Claim 21 metered in an amount between 500 ppm and 1000 ppm per litre of final product.

23. Use of a stabilising agent-free solubilizate as claimed in any one of Claims 1 to 9 as a preserving additive in dairy products such as milk, cheese or yoghurt, wherein the solubilizate is mainly applied to cheese rind.

24. Use of a stabilising agent-free solubilizate as claimed in any one of Claims 1 to 9 for preserving vegetables, fruit and herbs, expediently by immersing the vegetables, fruit or herbs in an aqueous solution of the solubilizate.

25. Use of a stabilising agent-free solubilizate as claimed in any one of Claims 1 to 9 for preserving surfaces of wood or synthetic materials, e.g., furniture, or surfaces of medical apparatuses and devices by means of treatment with a diluted, aqueous solution of the solubilizate.

## Revendications

1. Solubilisat sans agent stabilisant d'un conservateur destiné à permettre la conservation de produits alimentaires contre une altération microbienne, contenant un acide aliphatique et/ou un acide aromatique tels que, par exemple, l'acide sorbique et/ou l'acide benzoïque, de même qu'un ou plusieurs émulsifiants ayant une valeur d'équilibre hydrophile/lipophile comprise entre 9 et 18 avec une teneur en émulsifiant comprise entre environ 50 % en poids et environ 95 % en poids, par rapport à la quantité totale du solubilisat, l'acide étant présent sous forme micellaire, de telle sorte que l'acide libéré des micelles puisse agir directement sur les microorganismes en les détruisant, lorsque les micelles s'ouvrent au contact des membranes cellulaires ou d'une matière biologique similaire.

2. Solubilisat suivant la revendication 1, ayant une teneur en acide aliphatique et/ou en acide aromatique comprise entre 5 % en poids et 50 % en poids.

3. Solubilisat suivant la revendication 1 ou 2, dans lequel l'émulsifiant est un Polysorbate, principalement le Polysorbate 20 et/ou le Polysorbate 80.

4. Solubilisat suivant l'une des revendications précédentes, constitué d'environ 5 % en poids d'acide sorbique et d'environ 95 % en poids de Polysorbate 20.

5. Solubilisat suivant l'une des revendications 1 à 4, constitué d'environ 20 % en poids d'acide benzoïque et d'environ 80 % en poids de Polysorbate 20.

6. Solubilisat suivant l'une des revendications 1 à 4, constitué d'environ 20 % en poids d'acide benzoïque, environ 5 % en poids d'une huile contenant environ 5 % en poids d'un triglycéride principalement de moyenne longueur de chaîne, environ 73,5 % en poids de Polysorbate 20 et environ 1,5 % en poids d'une cire.

7. Solubilisat suivant la revendication 6, dans lequel la cire est de la cire d'abeille par exemple sous forme de cire blanche (Cera alba).

8. Solubilisat suivant la revendication 6 ou 7, dans lequel le Polysorbate 20 est remplacé par du Polysorbate 80.

9. Solubilisat suivant l'une des revendications 1 à 3, constitué d'environ 5 % en poids d'acide sorbique, environ 5 % en poids d'acide benzoïque et environ 90 % en poids de Polysorbate 20.

10. Procédé de production d'un solubilisat selon l'une ou plusieurs des revendications précédentes, dans lequel un émulsifiant ayant une valeur d'équilibre hydrophile/lipophile de 9-18 est chauffé à une température d'environ 70°C à environ 90°C, l'acide aliphatique et/ou l'acide aromatique est incorporé sous agitation à l'émulsifiant chaud sans refroidissement important de ce dernier, le mélange après l'incorporation totale est chauffé à une température d'environ 83°C à environ 90°C et convenablement homogénéisé sous agitation constante, puis refroidi, de telle sorte que la dissociation de l'acide ou des acides organiques soit ralentie ou empêchée par formation de micelles .

11. Procédé suivant la revendication 10, dans lequel une proportion d'environ 50 % en poids à environ 95 % en poids d'émulsifiant est chauffée.

12. Procédé suivant la revendication 10 ou 11, dans lequel un Polysorbate, principalement du Polysorbate 20 et/ou du Polysorbate 80, est utilisé comme émulsifiant.

13. Procédé suivant la revendication 12, dans lequel le Polysorbate est chauffé à une température d'environ 72°C à environ 85°C, avantageusement à une température d'environ 80°C à environ 85°C.

14. Procédé suivant l'une des revendications 10 à 13, dans lequel une proportion d'environ 5 % en poids à environ 50 % en poids d'un acide aliphatique et/ou d'un acide aromatique est incorporée à l'émulsifiant chaud.

15. Procédé suivant l'une des revendications 10 à 14, dans lequel un refroidissement rapide à environ 40°C est effectué.

16. Procédé suivant l'une des revendications 10 à 15, dans lequel de l'acide benzoïque est ajouté à un agent mouillant chaud et homogénéisé et le mélange est homogénéisé, après quoi environ un cinquième de la quantité de Polysorbate est ajouté au mélange d'acide benzoïque et d'agent mouillant et la masse obtenue est chauffée sous agitation et homogénéisée, puis la quantité de Polysorbate restante est ajoutée lentement et le mélange est agité à température élevée.

17. Procédé suivant la revendication 16, dans lequel l'agent mouillant est chauffé à une température d'environ 55°C à environ 65°C, avantageusement d'environ 58°C à environ 62°C.

18. Procédé suivant la revendication 15 ou 16, dans lequel la température élevée est choisie à une valeur d'environ 80°C à environ 90°C.

19. Procédé suivant l'une des revendications 16 à 18, dans lequel est choisi comme agent mouillant un mélange d'une huile contenant principalement des triglycérides à moyenne longueur de chaîne et d'une cire, avantageusement de la cire blanche (Cera alba).

20. Procédé suivant la revendication 19, dans lequel l'agent mouillant est formé par mélange d'environ 5 % en poids de l'huile et d'environ 1 % en poids à environ 1,5 % en poids de la cire.

21. Utilisation d'un solubilisat sans agent stabilisant suivant l'une des revendications 1 à 9 comme additif conservateur pour des produits alimentaires, en particulier pour des boissons sans alcool à haute valeur de pH allant par exemple d'environ 4,0 à environ 7,0 telles que des boissons à base de thé, de café, de cacao et de lait.

22. Utilisation suivant la revendication 21, selon un dosage compris entre 500 ppm et 1000 ppm par litre de produit final.

23. Utilisation d'un solubilisat sans agent stabilisant suivant l'une des revendications 1 à 9 comme additif conservateur pour produits laitiers tels que le lait, le fromage ou le yoghourt, le solubilisat étant principalement déposé sur de la croûte de fromage.

24. Utilisation d'un solubilisat sans agent stabilisant suivant l'une des revendications 1 à 9 pour la conservation de légumes, de fruits et d'herbes, convenablement par immersion de légumes ou de fruits ou d'herbes dans une solution aqueuse du solubilisat.

25. Utilisation d'un solubilisat sans agent stabilisant suivant l'une des revendications 1 à 9 pour la conservation de surfaces de bois ou de matières plastiques telles que, par exemple, des meubles, ou de surfaces d'appareils ou d'ustensiles médicaux par traitement avec une solution aqueuse diluée de solubilisat.
